# EUROPÄISCHE PATENTANMELDUNG

(11) **EP 3 599 232 A1**
(43) Veröffentlichungstag der Anmeldung: **29.01.2020**
(21) Anmeldenummer: 18185792.1
(22) Anmeldetag: 26.07.2018
(51) Int. Cl.: C07C 231/02, C07C 233/09, C08F 220/56

(54) **VERFAHREN ZUR HERSTELLUNG VON N-METHYL(METH)ACRYLAMID**

(71) Anmelder: Evonik Operations GmbH, 45128 Essen (DE)
(72) Erfinder: TRESKOW, Marcel, 64293 Darmstadt (DE); SAAL, Doris, 64625 Bensheim (DE); SCHÜTZ, Thorben, 64665 Alsbach-Hähnlein (DE); KRILL, Steffen, 64367 Mühltal (DE)
(74) Vertreter: Evonik Patent Association

(57) **Zusammenfassung**

Die Erfindung betrifft ein Verfahren zur Herstellung von N-Methyl(meth)acrylamid und deren Verwendungen.

## Beschreibung

Die Erfindung betrifft ein Verfahren zur Herstellung von N-Methyl(meth)acrylamid.

Die Herstellung von N-Methyl(meth)acrylamid kann durch Reaktion mit Säurehalogenid und Methylamin erfolgen. Hierbei wird aber ein zusätzliches Äquivalent Base benötigt, durch welchen der entstehende Halogenwasserstoff abgefangen wird. Im Zweifelsfall ist dies Methylamin selber, wodurch zwei Äquivalente Methylamin benötigt würden, wodurch die Wirtschaftlichkeit der Herstellung negativ beeinflusst wird.

Eine weitere Herstellung wird allgemein durch die DE4027843, ein kontinuierliches Verfahren zur Herstellung von N-substituierten Acryl- und Methacrylamiden, beschrieben. In diesem Verfahren werden durch Umsetzung von Alkylestern der Acryl oder Methacrylsäure mit aliphatischen oder aromatischen Aminen im Molverhältnis 1 ≥ 2 in kontinuierlicher Arbeitsweise, die entsprechenden N-substituierten Acrylsäureamide und Methacrylsäureamide gewonnen. Dieses Verfahren benötigt wie die Herstellung mit Säurechloriden zwei Äquivalente des Amins und ist daher nur bedingt atomökonomisch. Die Reaktion kommt zwar ohne Katalysator aus, erfolgt jedoch unter drastischen Bedingungen bei> 150°C und einem Druck von ca. 160 bar. Erfolgt die Herstellung von N-Methylmethacrylamid mit mehr Amin als der stöchiometrischen Methacrylsäureanhydrid Menge, so bildet sich ein nicht vakuumstabiles Salz. Bei der anschließenden Aufarbeitung zersetzt sich dieses, so dass das Vakuum nicht gehalten werden kann, die Temperatur unkontrolliert steigt und letztlich das Produkt polymerisiert.

DE102011089363 offenbart ebenfalls die Herstellung von N-Alkyl(alkyl)acrylamiden ebenfalls ausgehend vom entsprechenden Säureanhydrid und Alkylamin. Hier wird die Zugabe des Amins in wässriger Lösung beansprucht und die Lösung, vor Abtrennung des Produktes, neutralisiert. Für die Herstellung des N-Methyl(meth)acrylamid ist die Herstellung in wässriger Lösung sehr ungünstig. N-Methyl(meth)acrylamid ist eine Flüssigkeit, löst sich in Wasser in beliebigen Verhältnissen, lässt sich nur sehr schwer extrahieren und bei der destillativen Trennung kommt es aufgrund ähnlicher Siedepunkte zu sehr hohen Verlusten. Die Verwendung von wässrigen Lösungen des Amins, hat jedoch den entscheidenden Vorteil, dass das intermediär entstehende Ammoniumsalz der Säure in Anwesenheit von Wasser in ein Dissoziationsgleichgewicht gezwungen wird. Hierdurch sind immer geringe Spuren von Amin im System vorhanden die mit dem Anhydriden reagieren können, so dass die Gesamtausbeute >95% erreicht.

WO 2010/021956 offenbart die Herstellung von N-Alkyl(alkyl)acrylamiden ebenfalls ausgehend vom entsprechenden Säureanhydrid und Alkylamin. Hier wird die Zugabe des Anhydrids zum als wässrige Lösung vorgelegten Amin beansprucht. Diese Verfahrensvariante ist mit starker Wärmetönung verbunden, welche Verfärbungen verursachen kann. Darüber hinaus bestehen die identischen Nachteile wie in DE102011089363.

Aufgabe war es, ein besonders wirtschaftliches Verfahren zur Herstellung von N-Methyl(meth)acrylamid zur Verfügung zu stellen.

Die Aufgabe wurde gelöst durch ein Verfahren zur Herstellung von N-Methyl(meth)acrylamid durch Umsetzung von (Meth)acrylsäureanhydrid und Methylamin in Abwesenheit von Wasser. Insbesondere wurde die Aufgabe gelöst durch ein Verfahren zur Herstellung von N-Methyl(meth)acrylamid
dadurch gekennzeichnet, dass
a) (Meth)acrylsäureanhydrid mit Methylamin umgesetzt wird,
b) das Methylamin einen Wassergehalt <10 Gew% aufweist,
c) die Stöchiometrie von Anhydrid : Amin kleiner als 1:2 ist.

Überraschend wurde gefunden, dass mit dem erfindungsgemäßen Verfahren nahezu quantitative Umsätze erzielt werden und die Verluste bei Aufarbeitung zu einem Reinstoff besonders niedrig sind. Ferner kann das Nebenprodukt (Meth)acrylsäure wieder als Ausgangsstoff für die Herstellung des Anhydrids verwendet werden, so dass die Reaktion vollständig atomökonomisch und damit nicht nur besonders effizient sondern auch sehr nachhaltig ist.

Es wurde gefunden, dass das erfindungsgemäße Verfahren besonders vorteilhaft geführt werden kann, wenn die Umsetzung nahezu wasserfrei erfolgt.

Darüber hinaus wurde gefunden, dass die Reaktion ohne Katalysatorzugabe durchgeführt werden kann, was zu einer weiteren Verbesserung der Wirtschaftlichkeit des Verfahrens führt.

Die Schreibweise (Meth)acrylat bedeutet hier sowohl Methacrylat, wie z.B. Methylmethacrylat, Ethylmethacrylat usw., als auch Acrylat, wie z.B. Methylacrylat, Ethylacrylat usw., sowie Mischungen aus beiden.

### Katalysator

Die Reaktion kann in Abwesenheit von Katalysatoren durchgeführt werden. Die Reaktion ist ohne Katalysatorzugabe ausreichend schnell.

### (Meth)acrylsäureanhydrid

### Als (Meth)acrylsäureanhydrid werden Methacrylsäureanhydrid oder Acrylsäureanhydrid eingesetzt

### Stabilisator

Das (Meth)acrylsäureanhydrid wird bevorzugt stabilisiert eingesetzt. Geeignete Stabilisatoren sind: Phenotiazin, 2,4-Dimethyl-6-tert-butylphenol,N,N'-Diphenyl-p-phenylendiamin, 4-Hydroxy-2,2,6,6-tetramethylpiperidinooxyl, 4-Methyl-2,6-di-tert-butylphenol,2-tert-Butyl-6-(3-tert-butyl-2-hydroxy-5-methylbenzyl)-4-methylphenylacrylat, 1,4-Benzoldiamin, N,N'- gemischte Phenyl- und Tolylderivate (DTPD), 2,6-Di-tert-butyl-alpha-(dimethylamino)-p-cresol,tert-Butylbrenzkatechin, Bis-2,2,6,6-Tetramethyl-4-piperidylsebacat, 2,2,6,6-Tetramethylpiperidin-N-oxyl und deren Mischungen.

Die Stabilisatormenge wird zu Beginn der Reaktion als Lösung mit (Meth)acrylsäureanhydrid in den Reaktor eingetragen, vorgelegt oder nach dem (Meth)acrylsäureanhydrid zugegeben.

### Methylamin

Methylamin kann in flüssiger Form, gasförmig (beispielsweise Mono-Methylamin, Fa. GHC Gerling, Holz + Co, Hanau, Deutschland) oder in wasserfreien Lösungsmitteln, beispielsweise in THF, MTBE, Acetonitril, Chloroform, Dichlormethan oder Diethylether eingesetzt werden. Wässrige Methylamin-Lösungen sind für dieses Verfahren ungeeignet.

Bevorzugt beträgt der Wassergehalt des Methylamins < 10 Gew%, bevorzugt < 5 Gew%, besonders bevorzugt <1 Gew% und ganz besonders bevorzugt < 0,1 Gew%.

Bevorzugt beträgt der Wassergehalt des Reaktionsgemisches nach Ende der Zugabe aller Reaktanten < 10 Gew%, bevorzugt < 5 Gew%, besonders bevorzugt <1 Gew% und ganz besonders bevorzugt < 0,1 Gew%.

### Lösungsmittel

Die Umsetzung kann ohne Lösungsmittel, oder in Anwesenheit von Lösungsmitteln erfolgen. Geeignete Lösungsmittel sind ausgewählt aus der Gruppe: THF, MTBE, Diethylether, Dioxan, Acetonitril, Nitromethan, Chloroform, Dichlormethan, Benzol oder Toluol und deren Mischungen.

### Reaktionsbedingungen

Die Umsetzung erfolgt bei Temperaturen zwischen -20°C und 100°C, bevorzugt zwischen 0°C und 80°C und besonders bevorzugt zwischen 20°C und 50°C.

Zur Vermeidung der Bildung von unerwünschten Nebenprodukten wird bei besonders niedriger Temperatur gearbeitet um die Michael Addition von Aminen an die Doppelbindung des (Meth)acrylats zu unterbinden.

Die Umsetzung des (Meth)acrylsäureanhydrids mit Methylamin erfolgt bei einem absoluten Druck von 0,5 bar bis 10 bar, bevorzugt 1 bar bis 5 bar.

Die Reaktionsdauer liegt im Bereich von 0,1-10 h, bevorzugt 0,5-5 h.

### Stöchiometrie

Die Stöchiometrie von (Meth)acrylsäureanhydrid:Amin ist kleiner als 1:2, bevorzugt kleiner als 1:1,5, besonders bevorzugt kleiner als 1:1,1 und ganz besonders bevorzugt genau 1:1.

In einer weiteren Verfahrensausführung in welcher drucklos gearbeitet wird, kann Methylamin auch in die Reaktionslösung eingeperlt werden. Hierbei reagiert nicht der ganze Teil des Methylamins ab. Maßgeblich für das Gelingen der Reaktion, ist dass der Überschuss des reagierten Amins zu (Meth)acrylsäureanhydrid zwischen 0,5 bis 1,5 liegt.

### Neutralisation und Aufarbeitung:

Das erhaltene Rohprodukt kann direkt nach Ende der Reaktion mittels einer fraktionierten Destillation aufgearbeitet werden, ohne das eine Neutralisation erfolgen müsste oder sollte.

### Bevorzugte Verfahrensvarianten

Das (Meth)acrylsäureanhydrid wird in einem Autoklaven vorgelegt und der Autoklav verschraubt. Die Methylamin-Gasstahlflasche wird mit einem gewendeltem VA-Zuleitungsrohr mit dem Autoklaven verbunden, und die Gewichtsabnahme bei der Methylamineinleitung überwacht.

Die Einleitung von Methylamin wird mit einer Zulaufgeschwindigkeit von ca. 4g/3 min bei Raumtemperatur gestartet. Die Reaktion ist stark exotherm. Die Temperatur sollte nicht über 40°C steigen (35°C ± 5°C). Es wird mit einer Aceton Trockeneis Mischung gekühlt. Die Dosierung wird auf 6 g/3 min Methylamin erhöht. Nachdem die stöchiometrische Menge an Methylamin eingeleitet wurde, wurde die Flasche zugedreht, das restliche Gas in der Leitung 10 min abreagieren gelassen und anschließend die Kühlung entfernt und der Autoklav entlüftet.

Ein alternatives Verfahren ist die Umsetzung in Anwesenheit von Lösungsmitteln. (Meth)acrylsäureanhydrid und ein geeignetes Lösungsmittel, beispielsweise MTBE, werden vorgelegt und abgekühlt. Bei ca. 2°C - 10°C wird das Methylamin-Gas eingeleitet. Die Reaktion ist schwach exotherm.

Da die Reaktion bei dieser Temperatur nur sehr langsam abläuft, kann gegebenenfalls kräftiger Gas eingeleitet werden. Dabei kann die Sumpftemperatur auf bis zu 50°C steigen. Die Einleitung wird beendet, wenn kein (Meth)crylsäureanhydrid mehr nachweisbar ist. Das Lösungsmittel wird, beispielsweise mit einem Rotationsverdampfer, abgezogen. Der Rückstand wird destilliert.

Die erfindungsgemäß hergestellten N-Methyl(meth)acrylamide finden Anwendung als Copolymer in (Meth)acrylatpolymeren, um die Wasserlöslichkeit eines Polymers zu erhöhen.

Die im Folgenden gegebenen Beispiele werden zur besseren Veranschaulichung der vorliegenden Erfindung gegeben, sind jedoch nicht dazu geeignet, die Erfindung auf die hierin offenbarten Merkmale zu beschränken.

Das in den vorliegenden Beispielen verwendete Methacrylsäureanhydrid ist immer mit 2000 ppm 2,4-Dimethyl-6-tert-butylphenol stabilisiert.

### Beispiel 1: Darstellung von N-Methylmethacrylamid

### Reaktionsgleichung:

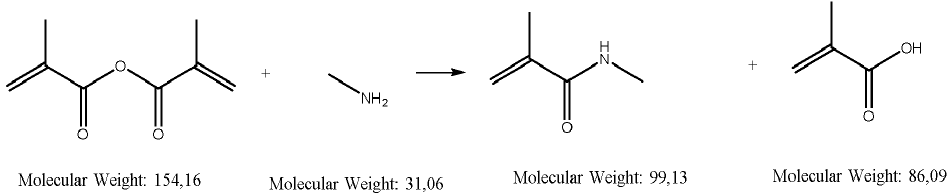

**Apparatur:** 2 L Autoklav mit Glaseinsatz, Ni-Cr-Ni-Thermoelement, Gaszuleitung Metall, Methylamin-Gasstahlflasche, Manometer, Aceton-Trockeneis-Bad

### Ansatz:

| | | | | |
|---|---|---|---|---|
| 6,0 | mol | Methacrylsäureanhydrid | = | 940,2 g |
| 6,0 | mol | Methylamin, Gas | = | 186,4 g |

**Th. Ausbeute:** (= Einwaage) 1126,6 g

**Durchführung:** Das Methacrylsäureanhydrid wird im Glaseinsatz des Autoklaven vorgelegt und der Autoklav verschraubt. Die Methylamin-Gasstahlflasche steht auf einer Waage und wird mit einem gewendeltem VA-Zuleitungsrohr mit dem Autoklaven verbunden, damit die Gewichtsabnahme bei der Methylamineinleitung überwacht werden kann.

Die Einleitung von Methylamin mit einer Dosiergeschwindigkeit von ca. 4g/3 min bei Raumtemperatur gestartet. Die Reaktion ist stark exotherm. Die Temperatur sollte nicht über 40°C steigen (35°C ±5°C). Es wird mit einer Aceton Trockeneis Mischung gekühlt. Die Dosierung wird auf 6 g/3 min Methylamin erhöht, mehr ist durch die Kühlung infolge des schlechten Wärmeübergangs wegen des Glaseinsatzes nicht möglich. Nachdem die stöchiometrische Menge an Methylamin (186,4 g, 6 mol) eingeleitet wurde, wird die Flasche zugedreht, das restliche Gas in der Leitung 10 min abreagieren gelassen und anschließend die Kühlung entfernt und der Autoklav entlüftet.

### Ausbeute: 1125 g

GC des Produkts, Methode : (GC: ,DB5, 30 m, Ø 0,25 mm, Filmdicke 0,25 µm, 50°C, 3 min isotherm, 14°C/min ->280°C, 8 min isotherm; Det: 280°C, Inj.: 250°C))

| Name | Methylamin | Methacrylsäure | N-Methylmethacrylamid | Methacrylsäureanhydrid | HS |
|---|---|---|---|---|---|
| Retention: | 1,08 min | 3,83 min | 5,97 min | 7,43 min | 13,15 min |
| GC-FI% | 0,045 | 41,360 | 52,445 | 0,079 | 4,187 |

| | | | | | |
|---|---|---|---|---|---|
| HS = Hochsieder | | | | | |

### Beispiel 2: Aufarbeitung von N-Methylmethacrylamid durch Destillation

**Apparatur:** 2 L Dreihalsrundkolben mit Siedekapillare, Pt100-Temperaturfühler, 30 cm Spiegelkolonne mit Raschigringen 8 x 8, autom. Kolonnenkopf (Flüssigkeitsteiler) Rückflusskühler, Schlangenkühler, Thiele-Anschütz-Vorstoß, Vorlagen, Ölbad, Vakuumpumpe, Druckmessgerät

### Ansatz:

| | | | |
|---|---|---|---|
| 1122 | g | N-Methylmethacrylamid aus Beispiel 1 | |
| 22,4 | mg | 4-Hydroxy-2,2,6,6-tetramethylpiperidinooxyl (rad.) | (20 ppm) |
| 224,4 | mg | Hydrochinonmonomethylether | (200 ppm) |
| 1122 | mg | Octadecyl-3-(3,5-di-tert-butyl-4-hydroxyphenyl)-propionat | (1000 ppm) |

### Durchführung:

| Bad °C | Sumpf °C | Kopf °C | Druck mbar | Bemerkung |
|---|---|---|---|---|
| 28 | 28 | 28 | 13,0 | Beginn Beheizung |
| 108 | 88,9 | 50 | 4,0 | Siedet, RV 1:5; 1. Fraktion |
| 111 | 99,2 | 58 | 3,8 | 2.Fraktion |
| 112 | 100,4 | 62 | 3,8 | 3. Fraktion |
| 128 | 114,7 | 69 | 2,9 | 4. Fraktion |
| 134 | 119,6 | 89 | 2,8 | 5. Fraktion |
| 136 | 113 | 91 | 2,9 | RV 1:10 |
| 141 | 118 | 91 | 2,8 | RV 1:5 |
| 146 | 122 | 94 | 2,8 | 6. Fraktion |
| 147 | 122,6 | 94 | 2,7 | 7. Fraktion, |
| 131 | 104 | 77 | 2,4 | 8. Fraktion |
| 138 | 105,9 | 79 | 2,3 | 9. Fraktion |
| 139 | 106,4 | 79 | 2,3 | 10. Fraktion |
| 144 | 108,5 | 79 | 2,3 | RV 1:2,5 |
| 144 | 108,0 | 80 | 2,3 | RV 1:1 |
| 143 | 110,4 | 81 | 2,3 | 11. Fraktion |
| 153 | 130 | 77 | 2,3 | Ende |

| | | | | |
|---|---|---|---|---|
| RV = Rücklaufverhältnis | | | | |

**Ausbeute und Analysen GC:**

| Name | Menge | MethylAmin | MethacrylSäure | N-Methyl-Methacryl -amid | Methacryl säureanhydrid | HS 2 | HS 3 | HS 1 |
|---|---|---|---|---|---|---|---|---|
| Retention: [min] | g | 1,08 min | 3,83 min | 5,97 min | 7,43 min | 8,71 | 10,89 | 13,15 |
| Fraktion | | | | | | | | |
| 1. | 135,7 | 0,003 | 99,867 | 0,124 | | | | |
| 2. | 139,1 | 0,007 | 99,993 | | | | | |
| 3. | 84,2 | 0,004 | 99,938 | 0,037 | | | | |
| 4. | 50,4 | | 97,275 | 1,758 | 0,008 | 0,77 | | |
| 5. | 58,0 | 0,018 | 62,601 | 33,538 | 0,007 | 3,44 | | |
| 6. | 28,8 | | 38,609 | 56,422 | 0,008 | 4,52 | | |
| 7. | 30,4 | | 22,935 | 72,339 | 0,005 | 4,30 | | |
| 8. | 73,3 | | 7,300 | 90,296 | | 2,12 | | |
| 9. | 38,5 | | 3,850 | 94,531 | | 1,15 | 0,011 | |
| 10. | 230,3 | | 0,929 | 98,566 | | 0,36 | 0,060 | 0,011 |
| 11. | 113,6 | | 0,116 | 99,120 | | 0,23 | 0,339 | 0,053 |
| Kältefalle 1 | 8,5 | 0,004 | 99,921 | 0,067 | | | | |
| Kältefalle 2 | 1,5 | 0,027 | 65,237 | 30,016 | | 3,97 | | |
| Rückstand * | 75,6 | | 1,285 | 14,950 | | | 3,100 | 20,60 |

| | | | | | | | | |
|---|---|---|---|---|---|---|---|---|
| * dickflüssig, weitere Peaks: 11,257 min: 25,653 % ; 11,688 min: 15,458%; 12,259 min 5,177 % + weitere, kleinere Peaks | | | | | | | | |

### Beispiel 3: Darstellung von N-Methylmethacrylamid in Lösemittel

**Apparatur:** 1 L Vierhalsrundkolben mit KPG-Rührung (Teflonhülse), Pt100-Temperaturfühler, Gasleinleitung (Teflon), Gaszuleitung aus Teflon mit Waschflaschen als Sicherheitsflaschen, Rückflusskühler, Methylamin-Gasstahlflasche, Abluftleitung direkt in den Abzug, Zuleitung für Druckluft als Beiluft, Aceton-Trockeneis-Kühlbad

### Ansatz:

| | |
|---|---|
| 1,0 mol | Methacrylsäureanhydrid = 156,4g |
| 250 ml | Methyl-tert-Butylether (MTBE) |
| 1,0 mol | Methylamin, Gas |

**Th. Ausbeute: 99,1 g**

**Durchführung:** Methacrylsäureanhydrid und MTBE werden vorgelegt und abgekühlt. Bei ca. 2°C - 10°C wird das Methylamin-Gas eingeleitet. Die Reaktion ist schwach exotherm.
Da die Reaktion bei dieser Temperatur nur sehr langsam erfolgt, wird kräftiger Gas eingeleitet und die Sumpftemperatur lässt man auf bis zu 50°C steigen. Die Einleitung wird beendet, wenn kein Methacrylsäureanhydrid mehr nachweisbar ist. Das MTBE wird am Rotationsverdampfer (RVD) bei einer Badtemperatur von 60°C und einem Druck bis 200 mbar abgezogen.
Destillat bis 200 mbar: 151,5 g
Rückstand bis 200 mbar181,7 g

Der Rückstand (180 g) wird über eine 15 cm Vigreux-Kolonne im Vakuum unter Zusatz von 1000 ppm Octadecyl-3-(3,5-di-tert-butyl-4-hydroxyphenyl)-propionat, 500 ppm Hydrochinonmonomethylether und 20 ppm 4-Hydroxy-2,2,6,6-tetramethylpiperidinooxyl (rad.) destilliert.

| Bad [°C] | Sumpf [°C] | Kopf [°C] | Druck mbar | Bemerkung | Masse |
|---|---|---|---|---|---|
| 24 | 24 | 24 | 3,8 | Beginn Beheizung | |
| 83 | 64,7 | 53 | 2,1 | 1. Fraktion | 62,9 g |
| 85 | 70,5 | 64 | 1,8 | 2. Fraktion | 35,3 g |
| 90 | 72,3 | 69,0 | 1,7 | 3. Fraktion | 45,0 g |
| 92 | 75,2 | 71 | 1,6 | 4. Fraktion | 19,7g |
| 103 | 84,3 | 70 | 1,6 | Thermometer taucht nicht mehr ein | |
| 111 | 96,9 | 50 | 1,6 | Ende | 13,5g |

**Analysen:** (GC: ,DB5, 30 m, Ø 0,25 mm, Filmdicke 0,25 µm, 50°C, 3 min isotherm, 14°C/min - >280°C, 8 min isotherm; Det: 280°C, Inj.: 250°C))

| **Name** | MTBE | Methylamin | Methacryl-Säure | N-Methylmeth -acrylamid | MethacrylsäureAnhydrid | HS 1 |
|---|---|---|---|---|---|---|
| **Ret. [min]** | 1,33 | 1,08 | 3,83 | 5,97 min | 7,43 min | 13,15 |
| **Probe:** | | | | | | |
| Rkt: 1,5h | * | | 3,38 | 2,96 | 89,70 | 2,18 |
| Rkt: 3 h | * | | 14,82 | 16,23 | 59,33 | 4,19 |
| Rkt: 5 h | * | | 37,09 | 45,68 | 9,53 | 3,59 |
| Rkt: 5,5 h | * | | 40,80 | 51,43 | 0,05 | 3,05 |
| RVD Destillat | 98,63 | | 1,27 | 0,11 | | |
| RVD Rückst. | | 0,06 | 39,95 | 53,65 | 0,01 | 3,87 |
| Dest. 1. Fr | 0,02 | 0,02 | 85,52 | 14,26 | 0,01 | 0,02 |
| Dest. 2. Fr | | 0,04 | 37,91 | 61,80 | | 0,10 |
| Dest. 3. Fr | 0,03 | 0,05 | 10,45 | 88,92 | | 0,29 |
| Dest. 4. Fr | | 0,05 | 1,67 | 96,71 | | 0,93 |

| | | | | | | |
|---|---|---|---|---|---|---|
| * Nicht berücksichtigt bei Integration | | | | | | |

**Ausbeute über GC-Analysen:** 89,9 g N-Methylmethacrylamid = 90,7 % d. Th

### Vergleichsbeispiel 1: Herstellung von N-Methylmethacrylamid bei Überschuss Methylamin-Gas

### Apparatur: 1 L Vierhalsrundkolben mit KPG-Rührung (Teflonhülse), Pt100-Temperaturfühler, Gaszuleitung aus Teflon mit Waschflaschen als Sicherheitsflaschen, Gasleinleitung (Teflon), Rückflusskühler, Methylamin-Gasstahlflasche, Abluftleitung direkt in den Abzug, Zuleitung für Druckluft als Beiluft, Aceton-Trockeneis-Kühlbad

### Ansatz:

| | |
|---|---|
| 1,0 mol | Methacrylsäureanhydrid = 156,4 g |
| 250 ml | Methyl-tert-Butylether |
| ∼2 mol | Methylamin, Gas |

**Th. Ausbeute N-Methylmethacrylamid: 99,1 g**

**Durchführung:** Methacrylsäureanhydrid und MTBE wird vorgelegt und Methylamin-Gas wird unter kräftigem Rühren eingeleitet. Der Umsatz wird durch GC überwacht. Wenn kein Methacrylsäureanhydrid mehr nachzuweisen ist, wird trotzdem weiter Methylamin eingeleitet, da noch eine exotherme Reaktion stattfindet. Sobald diese schwächer wird, wird die Einleitung beendet. Nach dem Abstellen der Rührung bilden sich 2 Phasen. Das MTBE wird am Rotationsverdampfer bei 60°C Badtemperatur und einem Druck bis 200 mbar abgezogen.

| | |
|---|---|
| Destillat bis 200 mbar: | 177,1 g |
| Rückstand bis 200 mbar | 215,0 g |

| | MTBE 1,328 min | Methylamin 1,08 min | MethacrylSäure 3,83 min | N-Methylmeth -acrylamid 5,97 min | MethacrylsäureAnhydrid 7,43 min |
|---|---|---|---|---|---|
| 1h 5 min | * | | 10,35 | 11,15 | 73,14 |
| 2h 15 min | * | | 20,19 | 23,34 | 49,91 |
| 3h 15 min | * | | 27,59 | 32,88 | 32,24 |
| 4h 15 min | * | 1,70 | 40,17 | 51,52 | - |
| 5h 15 min | * | 5,98 | 37,83 | 46,93 | - |
| Untere Phase | * | 8,67 | 35,92 | 45,36 | |
| Obere Phase | * | 10,55 | 11,36 | 68,47 | |
| Destillat bis 200 mbar | 98,01 | 1,99 | | | |
| Rückstand bis 200 mbar | | 7,36 | 36,33 | 46,47 | |
| Destillation 1. Fraktion | | 2,42 | 34,07 | 63,32 | |

| | | | | | |
|---|---|---|---|---|---|
| * Nicht berücksichtigt | | | | | |

180 g des Rotationsverdampfer-Rückstands wird über eine 15 cm Vigreux-Kolonne im Vakuum unter Zusatz von 1000 ppm Octadecyl-3-(3,5-di-tert-butyl-4-hydroxyphenyl)-propionat, 500 ppm Hydrochinonmonomethylether und 20 ppm 4-Hydroxy-2,2,6,6-tetramethylpiperidinooxyl (rad.) destilliert.
Aufgrund der Zersetzung von Methylammoniummethacrylat ist der Druck ab einer Supftemperatur von 40°C nicht aufrecht zu erhalten. Ab 90°C Sumpftemperatur bildet sich Nebel in der Vorlage und der Apparatur. Das ist ein klares Zeichen der Reaktion eines Amins mit Säure. Unmittelbar nach Erhalt eines Destillats bildet sich ein Feststoff im Sumpf und der Ansatz polymerisiert innerhalb 10 min durch.

| Zeit | Bad °C | Sumpf °C | Kopf °C | Druck mbar | Bemerkung |
|---|---|---|---|---|---|
| 10:24 | 25 | 25 | 25 | 1,6 | Beginn Beheizung |
| 10:37 | 81 | 60,5 | 27 | 7,4 | |
| 10:58 | 117 | 92,2 | 45 | 13,1 | |
| 10:59 | 121 | 94,7 | 59 | 13,0 | Nebel in Vorlage |
| 11:01 | 121 | 97,5 | 80 | 13 | 1. Fraktion, Brocken im Sumpf |
| 11:03 | 123 | 95,6 | 71 | 4,1 | |
| 11:10 | 129 | 89,7 | 68 | 2,5 | Abgestellt, Sumpf polymer |

Ausbeute: Fraktion 1: 16,2 g

### Vergleichsbeispiel 2: Darstellung von N-Methylmethacrylamid aus wässrigen Methylamin

### Apparatur: 2-L Vierhalsrundkolben mit KPG-Säbelrührer, 500 ml Tropftrichter, Pt-100 Sumpfthermometer, Rückflusskühler, Kühlbad

### Ansatz:

| | | |
|---|---|---|
| 6,15 mol | Methacrylsäureanhydrid | = 961,5 g |
| 6,15 mol | Methylamin, 40% in H₂O | = 477,4 g |

**Th. Ausbeute:** 609,65g

### Durchführung:

Methacrylsäureanhydrid wird vorgelegt und unter Rühren auf unter 10°C abgekühlt. Dann wird die Dosierung von Methylamin gestartet (exotherme Reaktion, Kühlung Eisbad und leichte Nebelbildung). Es wird so zugetropft und gekühlt, dass die Temperatur nicht über 10°C steigt. Nach Zutropfende (4h) wird zur Nachreaktion noch 2 h weiter gerührt, die Kühlung wird dabei entfernt.

### Aufarbeitung:

Der Ansatzes (1419 g) wird am Rotationverdampfer unter Zusatz von 1000 ppm Octadecyl-3-(3,5-di-tert-butyl-4-hydroxyphenyl)-propionat, 500 ppm Hydrochinonmonomethylether und 20 ppm 4-Hydroxy-2,2,6,6-tetramethylpiperidinooxyl (rad.) eingeengt.

| | |
|---|---|
| Rückstand im Kolben: | 229,7 g |
| Destillat bis 35 mbar, Bad 80°C | 340,8 g |
| Destillat bis 5 mbar, Bad 80°C | 273,4 g |
| Destillat bis 0 mbar, Bad 80°C - 95°C | 531,6 g |

GC-Analytik: (DB5, 30 m, Ø 0,25 mm, Filmdicke 0,25 µm, 50°C, 3 min isotherm, 14°C/min - >280°C, 8 min isotherm; Det: 280°C, Inj.: 250°C))

| | Masse | Wassergehalt | MethacrylSäure | N-Methyl-Methacryl amid | HS1r | Masse Produkt isoliert |
|---|---|---|---|---|---|---|
| | | | [GC-FI%] | [GC-FI%] | [GC-Fl%] | |
| | | [g] | 4,73 min | 5,986 min | 13,174 min | [g] |
| Destillat bis 35 mbar | 340,8 | 180 | 92,93 | 6,83 | | 11,0 |
| Destillat bis 5 mbar | 273,4 | 83 | 81,02 | 18,74 | 0,03 | 35,7 |
| Destillat bis 1 mbar | 531,6 | 20 | 27,36 | 71,06 | 0,84 | 363,5 |
| SUMME | | | | | | 410,2 |

Ohne Neutralisation der wässrigen Phase, können nach einfacher Destillation 410,2g (67,3 % d. Theorie) des Produktes als Mischung mit Wasser und Methacrylsäure isoliert werden, es verbleibt ein Feststoff von rund 230g. Die Destillate bis 5mbar und bis 1mbar (805 g, mit 399g Produkt) werden im Vergleichsbeispiel 3 fraktioniert destilliert.

### Vergleichsbeispiel 3: Destillation von N-Methylmethacrylamid aus wässriger Methacrylsäure..

### Apparatur: 1 l Dreihalsrundkolben, Siedekapillare, 30 cm Spiegelkolonnen mit Raschigringen 6 x 6, Liebigkühler, Claisenaufsatz, Thiele-Anschütz-Vorstoß, Vorlage, Ölbad, Vakuumpumpe

**Ansatz:** 805 g Destillat aus Vergleichsbeispiel 2
66,5 mg Hydrochinonmonomethylether (100 ppm)
665 mg Octadecyl-3-(3,5-di-tert-butyl-4-hydroxyphenyl)-propionat (1000 ppm)
33,3 mg 2,4-Dimethyl-6-tert-butylphenol (50 ppm)

### Durchführung:

| Zeit | Bad °C | Sumpf °C | Kopf °C | Druck mbar | Bemerkung |
|---|---|---|---|---|---|
| 00:00 | 20 | 12,0 | 20 | 0,52 | Beginn Beheizung |
| 00:10 | 98 | 40,7 | 27 | 0,56 | 1. Fraktion |
| 00:14 | 97 | 50,3 | 30 | 0,72 | |
| 00:19 | 93 | 60,8 | 32 | 2,0 | Sumpf hat Feststoffanteil |
| 00:25 | 96 | 68,4 | 52 | 1,1 | |
| 00:52 | 96 | 69,1 | 42 | 0,35 | |
| 01:22 | 112 | 72,3 | 37 | 0,20 | 2. Fraktion, viel Feststoff im Sumpf |
| 01:36 | 113 | 73,4 | 38 | 0,21 | Starke Siedeverzüge im Sumpf, abgestellt |

| | |
|---|---|
| 1. Fraktion | 156,3 g |
| 2. Fraktion | 48,5 g (trübes Destillat, "Mini-Bröckchen") |

Der Sumpf wird über eine Porzellannutsche filtriert. Filtrat -> 292,5 g ->GC Eine Probe des Nutschenkuchens wird in MeOH gelöst- -> 307g -> Polymer GC-Analytik: (Meth. F241,DB5, 30 m, Ø 0,25 mm, Filmdicke 0,25 µm, 50°C, 3 min isotherm, 14°C/min ->280°C, 8 min isotherm; Det: 280°C, Inj.: 250°C))

| | Masse | Wassergehalt | MethacrylSäure | N-MethylMethacrylamid | HS1 |
|---|---|---|---|---|---|
| | | | [GC-FI%] | [GC-FI%] | [GC-FI%] |
| | | [g] | 4,73 min | 5,986 min | 13,174 min |
| 1. Fraktion | 156,3 | 103 | 99,887 | 0,083 | |
| 2. Fraktion | 48,5 | - | 99,616 | 0,320 | |
| Filtrat Sumpf | 292,5 | - | 64,559 | 35,100 | 0,049 |

Während Wasser und Methacrylsäure weitestgehend isoliert werden können, polymerisiert das mit wässrigen Methylamin hergestellte Produkt, so dass nach 90 min Destillation nur noch rund 25% N-Methylmethacrylamid vorhanden sind.

### Vergleichsbeispiel 4: Darstellung von N-Methylmethacrylamid aus wässrigen Methylamin, Aufarbeitung durch Extraktion

### Apparatur: Analog Vergleichsbeispiel 2

### Ansatz:

| | | | | | |
|---|---|---|---|---|---|
| 2,5 | mol | F49 (18770-45, 96,61%; 1000 ppm S49) | = | 398,9 | g |
| 2,5 | mol | N-Methylamin, 40% in H₂O | = | 194,1 | g |
| 2,5 | mol Ammoniak, 25% | | = | 170 | g |

**Th. Ausbeute: 247,8 g**

### Durchführung: Herstellung des Rohproduktes analog Vergleichsbeispiel 2

Das Rohprodukt wird mit NH₃- Lösung neutralisiert, dabei wird teilweise gekühlt. Es erfolgte keine Phasentrennung.
Der Ansatz wird anschließend mit MTBE extrahiert (laut GC nur Methacrylsäure enthalten). Anschließend wird 3 x mit je 300 ml Methylmethacrylat (MMA) und danach erneut 3 x mit je 200 ml MMA extrahiert (im letzten Extrakt sind noch 4 % Produkt enthalten). Die vereinigten MMA-Phasen werden am Rotationsverdampfer im Vakuum eingeengt (Badtemperatur 60°C), dabei wird noch vorhandenes H₂O mitgeschleppt.
**Ausbeute:** 115,8 g
GC-Analytik: 20,4% Methacrylsäure, 69,28% N-Methylmethacrylamid
Die Systeme Wasser/Methacrylsäure und N-Methylmethacrylamid sind sich sehr ähnlich. Eine Extraktion kann nur mit viel Aufwand und schlechter Ausbeute eine Abtrennung des Produktes ermöglichen.

### Vergleichsbeispiel: 5:Darstellung von N-Methylmethacrylamid durch wässriges Methylamin und Neutralisation des Rückstandes mit KOH

### Apparatur: 2-L Vierhalsrundkolben mit KPG-Säbelrührer, 500 ml Tropftrichter, Pt-100 Sumpfthermometer, Rückflusskühler, Eisbad

### Ansatz:

| | |
|---|---|
| 6,0mol | Methacrylsäureanhydrid = 938,1 g |
| 6,0 mol | Methylamin, 40% in H₂O = 465,9 g* |

**Th. Ausbeute:** 594,8 g

**Durchführung:** Methacrylsäureanhydrid wird vorgelegt und auf unter 10°C abgekühlt. Dann wird die Dosierung von Methylamin gestartet (exotherme Reaktion, Kühlung mit Eis/H₂O, leichte Nebelbildung) Es wird so zugetropft und gekühlt, dass die Temperatur nicht über 10°C steigt. Nach Zutropfende wird zur Nachreaktion noch 2 h weiter gerührt. Die Kühlung wird dabei entfernt. Nach Lagerung über Nacht (Ansatz wird dabei hell-gelb) wird der Ansatz in einen 4l Kolben überführt und mit 5,9 mol KOH (50%ig in H₂O, Zugabe über Tropftrichter, Kühlung mit H₂O-Bad, Zusatz von 2 Tropfen Phenolphthalein-Lösung) neutralisiert -> pH 6,5.
Der ausgefalle Feststoff wird dann über eine Porzellannutsche abgesaugt, das Filtat am Rotationsverdampfer im Vakuum (Bad bis 80°C, Druck siehe unten) unter Zusatz von 1000 ppm Octadecyl-3-(3,5-di-tert-butyl-4-hydroxyphenyl)-propionat, 500 ppm Hydrochinonmonomethylether und 20 ppm 4-Hydroxy-2,2,6,6-tetramethylpiperidinooxyl (rad.). (bez. auf th. Ausbeute N-Methylmethacrylamid) eingeengt.

| | | |
|---|---|---|
| Rückstand auf der Nutsche | 77,5 g | (b) |
| Rückstand im Kolben | 1026,2 g | (a) |

GC-Analytik: (DB5, 30 m, Ø 0,25 mm, Filmdicke 0,25 µm, 50°C, 3 min isotherm, 14°C/min - >280°C, 8 min isotherm; Det: 280°C, Inj.: 250°C))

| | Masse | NS | MethacrylSäure | N-Methyl-Methacryl amid | HS1 | Anteil Produkt in Fraktion | Masse Produkt isoliert |
|---|---|---|---|---|---|---|---|
| | [g] | | [GC-FI%] | [GC-FI%] | [GC-FI%] | [Gew%] | [g] |
| | | 1,09-1,20 min | 4,73 min | 5,986 min | 13,174 min | | |
| Dest. bis 60 mbar | 316,4 | 1,081 | 6,121 | 89,158 | 1,113 | 7,0 | 22,15 |
| Dest. bis 1 mbar | 163,8 | 1,110 | 8,088 | 90,161 | 0,090 | 94,5 | 154,05 |

| | | | | | | | |
|---|---|---|---|---|---|---|---|
| NS= Niedrigsieder | | | | | | | |

Zur Ermittlung der Masse des Produkts in der Fraktion wird eine genau definierte Menge Dimethylaminopropylmethacrylamid zugegegben und über den Vergleich der Integrale die genaue Masse bestimmt.
Bei Neutralisation der wässrigen Phase mit KOH können nach Destillation lediglich 144,9 g (29,6 % d. Theorie) des Produktes als verdünnte wässrige Lösung isoliert werden.

### Vergleichsbeispiel 6:Darstellung von N-Methylmethacrylamid durch wässriges Methylamin und Neutralisation des Rückstandes mit NaOH

### Apparatur: 2-L Vierhalsrundkolben mit KPG-Säbelrührer, 500 ml Tropftrichter, Pt-100 Sumpfthermometer, Rückflusskühler, Kühlbad

### Ansatz:

6,0 mol Methacrylsäureanhydrid = 938,1 g
6,0 mol Methylamin, 40% in H₂O = 465,9 g*
**Th. Ausbeute:** 594,8 g

**Durchführung:** Methacrylsäureanhydrid wird vorgelegt und auf unter 10°C abgekühlt. Dann wird die Dosierung von Methylamin gestartet (exotherme Reaktion, Kühlung mit Eis/H₂O, leichte Nebelbildung) Es wird so zugetropft und gekühlt, dass die Temperatur nicht über 10°C steigt. Nach Zutropfende wird zur Nachreaktion noch 2 h weiter gerührt, die Kühlung wird dabei entfernt.

Anschließend wird der Ansatz in einen 4l Kolben überführt und mit 5,9 mol NaOH 50%ig in H₂O, (236 g NaOH, 236 g H₂O*) durch Zugabe über Tropftrichter unter Kühlung mit H₂O-Bad und Zusatz von 2 Tropfen Phenolphthalein-Lösung neutralisiert bis auf pH 6,5 - pH 7,0.
Der ausgefallene Feststoff wird über eine Porzellan-Nutsche abgesaugt -> sehr schlechte Filtration, da wachsartiger Feststoff.

Filtrat: 1634 g

Zur Analyse wird im GC-Vial 1,5 g Filtrat mit einem internen Standard versetzt und hierüber der Gehalt an N-Methylmethacrylamid bestimmt:
Berechneter Produkt Gehalt nach GC-Analyse: 36,4 % -99,9% d. Theorie.

Das Filtrat wird am Rotationsverdampfer im Vakuum eingeengt (1). Das am Kühler kondensierte (vereiste) Kondensat wird über Nacht aufgetaut und am nächsten Tag der Rückstand-Kolben nochmals bei 80°C Badtemperatur und 1 mbar bis zur Trockne eingeengt (2). Hierbei fallen signifikante Mengen an Feststoff aus. Die erhaltenen Destillate werden mittels GC analysiert:
GC-: (DB5, 30 m, Ø 0,25 mm, Filmdicke 0,25 µm, 50°C, 3 min isotherm, 14°C/min ->280°C, 8 min isotherm; Det: 280°C, Inj.: 250°C))

| Probe | Masse [g] | Produktgehalt nach GC (wie in VB5 beschrieben). [Gew.%] | Produkt-Masse [g] |
|---|---|---|---|
| Filtrat | 1634 | 36,4 | 594,8 |
| Destillat bis 60 mbar | 172,2 | 14,0 | 24,1 |
| Destillat Kolben | 7,5 | 79,3 | 6,0 |
| vom Kühler abgetaut (1) | 230,6 | 23,1 | 53,3 |
| vom Kühler abgetaut (2) | 129,2 | 47,7 | 61,6 |
| Summe Destillate | 539,5 | | 144,9 |

Bei Neutralisation der wässrigen Phase mit NaOH können nach Destillation lediglich 144,9g (24,4 % d. Theorie) des Produktes als verdünnte wässrige Lösung isoliert werden.

## Patentansprüche

1. Verfahren zur Herstellung von N-Methyl(meth)acrylamid
**dadurch gekennzeichnet, dass**
a) (Meth)acrylsäureanhydrid mit Methylamin umgesetzt wird,
b) das Methylamin einen Wassergehalt <10 Gew.% aufweist,
c) die Stöchiometrie von (Meth)acrylsäureanhydrid : Amin kleiner als 1:2 ist.

2. Verfahren nach Anspruch 1, **dadurch gekennzeichnet, dass** der Wassergehalt des Methylamins kleiner 5 Gew.% ist, bevorzugt kleiner 1 Gew.% ist und ganz besonders bevorzugt < 0,1 Gew.% ist.

3. Verfahren nach Anspruch 1, **dadurch gekennzeichnet, dass** der Wassergehalt nach Ende der Zugabe aller Reaktanten kleiner 10 Gew.% ist.

4. Verfahren nach Anspruch 3, **dadurch gekennzeichnet, dass** der Wassergehalt nach Ende der Zugabe aller Reaktanten kleiner 5 Gew.% ist, bevorzugt kleiner 1 Gew.% ist und ganz besonders bevorzugt < 0,1 Gew.% ist.

5. Verfahren nach Anspruch 1, **dadurch gekennzeichnet, dass** die Reaktion in einem polaren, nicht protischen Lösemittel durchgeführt wird.

6. Verfahren nach Anspruch 1, **dadurch gekennzeichnet, dass** das Methylamin als Reinstoff, flüssig oder gasförmig, zugegeben wird.

7. Verfahren nach Anspruch 1, **dadurch gekennzeichnet, dass** Methylamin als Gas zugegeben wird.

8. Verfahren nach Anspruch 1, **dadurch gekennzeichnet, dass** der Überschuss des reagierten Amins zu (Meth)acrylsäureanhydrid zwischen 0,5 bis 1,5 liegt.

9. Verfahren nach Anspruch 1 bis 8, **dadurch gekennzeichnet, dass** eine Entfernung oder Abtrennung des Nebenproduktes (Meth)acrylsäure durch Destillation erfolgt.

10. Verfahren nach Anspruch 1 bis 9, **dadurch gekennzeichnet, dass** die Umsetzung des (Meth)acrylsäureanhydrids mit Methylamin bei Temperaturen zwischen -20°C und 100°C, bevorzugt zwischen 0°C und 80°C und besonders bevorzugt zwischen 20°C und 50°C erfolgt.

11. Verfahren nach Anspruch 1 bis 10, **dadurch gekennzeichnet, dass** die Umsetzung des (Meth)acrylsäureanhydrids mit Methylamin bei einem absoluten Druck von 0,5 bar bis 10 bar, bevorzugt 1 bar bis 5 bar erfolgt.

12. Verwendung von N-Methyl(meth)acrylamiden gemäß der Ansprüche 1-11 in Copolymeren.
